# FASCICULE DE BREVET EUROPEEN

(11) **EP 0 805 850 B1**
(45) Date de publication et mention de la délivrance du brevet: **15.12.1999**
(21) Numéro de dépôt: 96901854.8
(22) Date de dépôt: 25.01.1996
(51) Int. Cl.: C12N 1/20, C12P 19/04

(54) **NOUVELLE SOUCHE DE KLEBSIELLA PNEUMONIAE SUBsp. PNEUMONIAE ET PROCEDE DE PRODUCTION D'UN POLYSACCHARIDE CONTENANT DU L-FUCOSE**
NEUER STAMM VON KLEBSIELLA PNEUMONIAE,SUBSP.PNEUMONIAE UND VERFAHREN ZUR PRUDUKTION EINES L-FUCOSE ENTHALTENDEN POLYSACCHARIDS
NEW STRAIN OF KLEBSIELLA PNEUMONIAE, SUBSP. PNEUMONIAE AND PROCESS FOR PRODUCING A POLYSACCHARIDE CONTAINING L-FUCOSE

(30) Priorité: 26.01.1995 FR 9500898
(43) Date de publication de la demande: 12.11.1997
(73) Titulaire: BIOEUROPE, 28260 ANET (FR)
(72) Inventeur: PAUL, François, Marie, Bernard, F-31400 Toulouse (FR); PERRY, David, Frank, F-31100 Toulouse (FR); MONSAN, Pierre, Frédéric, F-31700 Blagnac (FR)
(74) Mandataire: Colas, Jean-Pierre
(86) Numéro de dépôt international: FR9600127
(87) Numéro de publication internationale: WO9623057

(56) Documents cités:
- US-A- 4 298 691
- CARBOHYDRATE RES., vol. 77, 1979, pages 183-190, XP002000740 JOSELEAU J-P. ET AL.: "Structure of the capsular polysaccharide of Klebsiella K-type 63" cité dans la demande

## Description

L'invention concerne une nouvelle souche de *Klebsiella pneumoniae,* subsp. *pneumoniae* ainsi qu'un procédé de production d'un polysaccharide contenant du L-fucose mettant en oeuvre cette souche.

La nouvelle souche de *Klebsiella pneumoniae* subsp. pneumoniae (appelée ci-après BEC 1000) de l'invention a été déposée le 16 Décembre 1994 à la Collection Nationale de Cultures de Microorganismes, 25, rue du Docteur Roux 75724 PARIS CEDEX 15, conformément aux provisions du Traité de Budapest. Elle a reçu le No. d'enregistrement I-1507.

L'utilisation de souches bactériennes du genre *Klebsiella* pour produire des polysaccharides contenant du L-fucose est connue.

J.P. JOSELEAU et M.-F. MARAIS, Carbohydrate Research, 77 (1979), pages 183-190 ont décrit un polysaccharide capsulaire contenant du D-galactose, du L-fucose et de l'acide D-galacturonique dans le rapport 1:1:1 provenant d'une souche de *Klebsiella* K-63.

G.T. VEEDER et K.S. KANG, US-A-4 298 691, ont décrit un procédé de production d'un hétéropolysaccharide S-156 par culture d'une souche de *Klebsiella pneumoniae,* voisine de la souche K-63 précédente, déposée sous le No. ATCC 31646 auprès de l'American Type Culture Collection le 12 Mai 1988, dans un milieu nutritif aqueux par fermentation aérobie d'une source de glucides assimilables, telle que de l'amidon hydrolysé. Cet hétéropolysaccharide contient de l'acide D-galacturonique, du D-galactose et du L-fucose dans un rapport molaire approximatif de 23:21:26.

A la suite de recherches poussées, les présents inventeurs ont trouvé une nouvelle souche de K. pneumoniae, subsp. pneumoniae, dite BEC 1000, qui s'est révélée capable de produire, avec des rendements industriellement utiles, un polysaccharide contenant du L-fucose, présentant des propriétés intéressantes dans le domaine cosmétique, telles que le toucher, le pouvoir hydratant, la capacité de stabiliser des émulsions, et le pouvoir de rémanence des parfums.

La souche BEC 1000 a été isolée à partir de boues d'une station d'épuration. Un échantillon de 1g de boue a servi à inoculer 10 ml de milieu de culture placés dans un erlenmeyer de 100 ml. Le milieu de culture contenait par litre, 0,02g d'extrait de levure, 10g de glucose, 9g de K₂HPO₄, 3g de KH₂PO₄, 1g de (NH₄)₂SO₄, et 0,2g de MgSO₄, 7H₂O, avait un pH de 7,2, et avait été traité à l'autoclave à 110°C pendant 30 mn au préalable. La culture a duré 6 jours à 30°C, sous agitation à 120 tours/mn. On a préparé une série de dilutions croissantes du milieu de culture et on les a chacune étalées sur du milieu de culture additionné de 2% en poids d'agar-agar dans une boîte de Pétri. Au bout de 2 jours, on a isolé les colonies qui présentaient un aspect mucoïde prononcé.

La souche de l'invention a été sélectionnée après remise en culture dans du milieu de culture des colonies fortement mucoïdes parce qu'elle donnait une réponse particulièrement positive aux dosages suivants :
- Dosage des 6-désoxyhexoses (méthylpentoses) selon la méthode spectrophotométrique de Z. DISCHE et L.B. SHETTLES: "A specific color reaction of methyl-pentoses and a spectrophotometric micromethod for their determination". J. Biol. Chem. 175, 595-603 (1948).
- Dosage du L-fucose par chromatographie couche mince (C.C.M.).
- Dosage du L-fucose par chromatographie liquide haute pression (HPLC).

La souche BEC 1000 de l'invention présente les caractéristiques suivantes :
IMAGES CELLULAIRES- Bacilles homogènes, sans arrangements notables
- Flagelles :: souche immobile
- GRAM :: coloration négative
CONDITIONS DE BASE : 30°, atmosphère ambiante
Description des cultures : Culture luxuriante, non pigmentée, sur géloses et en bouillons
Culture aéro-anaérobie en gélose profonde

| | | | |
|---|---|---|---|
| SALINITES (milieu de culture contenant par litre : peptone : 10 g, extrait de levure : 3 g, glucose : 5 g ; Ph 7,0 - 7,2, 30°C, aération et agitation) | | | |
| - NaCl 0% | 3 | 3 | 3 |
| - NaCl 2% | 3 | 3 | 3 |
| - NaCl 4% | 3 | 3 | 3 |
| - NaCl 6% | 3 | 3 | 3 |
| - NaCl 8% | 2 | 2 | 2 |
| - NaCl 10% | 0 | 1 | 1 |
| TEMPERATURES : (milieu Columbia, Ph 7,0 - 7,2, aération) | | | |
| - 5° | 0 | 0 | 0 |
| - 10° | 0 | 1 | 2 |
| - 15° | 1 | 2 | 3 |
| - 30° | 3 | 3 | 3 |
| - 41° | 3 | 3 | 3 |
| - 45° | 3 | 3 | 3 |
| Culture prototrophe | 3 | 3 | 3 |
| Milieu de culture contenant par litre : KH₂PO₄ : 3,9 g, K₂HPO₄: 11,2 g (NH₄)₂ SO₄ : 2 g, MgSO₄, 7H₂O : 200 mg, FeSO₄, 7H₂O : 1 ml d'une solution à 50 mg/l, Ph 7,1-7,3 | | | |
| - CATALASE | | 3 | |
| - REACTION D'OXYDASE | | 0 | |
| - NITRATE REDUCTASE | | 3 | 3 |
| - TETRATHIONATE REDUCTASE | | 0 | 0 |
| - THIOSULFATE REDUCTASE | | 0 | 0 |
| PRODUCTION ACIDE (milieu de culture contenant par litre : bactopeptone : 3 g, NaCl : 5 g, glucose : 10 g, pH 7,2-7,4, en présence de rouge de phénol) | | | |
| - tube ouvert | 3 | 3 | 3 |
| - tube fermé | 3 | 3 | 3 |
| REACTION VOGES-PROSKAUER | | | |
| Milieu de culture BHI (Brain Heart Infusion), pH 7,0-7,2: | | | |
| - Production de gaz (en mm) à partir de glucose | | 70 | |
| - UREASE | | 0 | 0 |
| - PRODUCTION D'INDOLE | | 0 | 0 |
| - Gamma-glutamyl transférase | | 0 | 0 |
| - Phénylalanine désaminase | | 0 | 0 |
| - Arginine dihydrolase | | | 0 |
| - Lysine décarboxylase | | | 3 |
| - Ornithine décarboxylase | | | 0 |
| - HYDROLYSE du o-nitrophényl-β-D-galactoside | | 3 | 3 |
| - HYDROLYSE du p-nitrophénylxyloside | | 3 | 3 |
| - HYDROLYSE d'ESCULINE | | 3 | 3 |
| - HYDROLYSE du TWEEN 80 | | 0 | 0 |
| - DNAse extracellulaire | | 0 | 0 |
| - Gélatinase (méthode de Frazier) | | 0 | 0 |
| CULTURE EN MILIEU SYNTHETIQUE : Milieu de culture contenant par litre : KH₂PO₄ : 3,9 g, K₂HPO₄ : 11 g (NH₄)₂ SO₄ : 2 g, MgSO₄, 7H₂O : 200 mg, FeSO₄,7H₂O : 1 ml d'une solution à 50 mg/l, aération et agitation | | | |
| - Citrate | 3 | 3 | 3 |
| - Glucose | 3 | 3 | 3 |
| - Hydroxy-3-benzoate | 0 | 0 | 0 |
| - Hydroxy-5-proline | 0 | 2 | 3 |
| - Malonate | 2 | 3 | 3 |
| Nota : les diverses colonnes de résultats correspondent à des temps de lecture différents, pouvant varier d'un essai à l'autre. Explication des symboles : 0 : réaction ou culture négative 1 : réaction positive faible 2 : réaction positive 3 : réaction positive intense | | | |

La souche BEC 1000 présente également le profil nutritionnel suivant. A titre comparatif, on a également mentionné le profil de la souche ATCC 31646, en signalant par une astérisque les substrats donnant une réponse différente.

**TABLEAU 1**

| Source de carbone | Croissance/réaction | |
|---|---|---|
| | BEC 1000 | ATCC 31646 |
| N-Acétyl-D-glucosamine | + | + |
| cis-Aconitate | + | + |
| trans-Aconitate | + | + |
| Adonitol* | + | - |
| D-Alanine | + | + |
| L-Alanine | + | + |
| 4-Aminobutyrate | + | + |
| 5-Aminovalérate | + | + |
| L-Arabinose | + | + |
| D-Arabitol | + | + |
| L-Arabitol | - | - |
| D-Aspartate | + | + |
| Benzoate | + | + |
| Bétaïne | - | - |
| Caprate | - | - |
| Caprylate | - | - |
| D-Cellobiose | + | + |
| Citrate | + | + |
| m-Coumarate* | - | + |
| Dulcitol | - | - |
| i-Erythritol | - | - |
| Esculine (couleur noire) | + | + |
| Ethanolamine* | + | - |
| D-fructose | + | + |
| L-Fucose | + | + |
| Fumarate | + | + |
| D-Galactose | + | + |
| D-Galacturonate | + | + |
| Gentiobiose | + | + |
| Gentisate | - | - |
| D-Gluconate | + | + |
| D-Glucosamine | + | + |
| D-Glucose | + | + |
| D-Glucuronate | + | + |
| L-Glutamate | + | + |
| Glutarate | - | - |
| DL-Glycérate* | + | - |
| Glycérol | + | + |
| Histamine | - | - |
| L-Histidine (couleur rose) | - | - |
| 3-Hydroxybenzoate | - | - |
| 4-Hydroxybenzoate | + | + |
| 3-hydroxybutyrate | + | + |
| HQ-β-glucuronide | - | - |
| myo-Inositol | + | + |
| Itacoate | - | - |
| 2-cétogluconate | + | + |
| 5-cétogluconate* | - | + |
| 2-cétoglutarate | - | - |
| DL-Lactate | + | + |
| Lactose | + | + |
| Lactulose | (+) | (+) |
| D-Lyxose | - | - |
| D-Malate* | - | + |
| L-Malate | + | + |
| Malonate* | + | - |
| Maltitol* | - | + |
| Maltose | + | + |
| Maltotriose | + | + |
| D-Mannitol | + | + |
| D-Mannose | + | + |
| D-Mélézitose | - | - |
| D-Mélibiose | + | + |
| 1-O-Méthyl-α-galactoside | + | + |
| 1-O-Méthyl-β-galactoside | + | + |
| 3-O-Méthyl-D-glucose | - | - |
| 1-O-Méthyl-α-D-glucoside | (+) | (+) |
| 1-O-Méthyl-β-D-glucoside | + | + |
| Mucate | + | + |
| Palatinose | + | + |
| Phénylacétate | + | + |
| 3-phénylpropionate | - | - |
| L-Proline | + | + |
| Propionate | - | - |
| Protocatéchuate | + | + |
| Putrescine | + | + |
| Quinate | + | + |
| D-Raffinose | + | + |
| L-Rhamnose | + | + |
| D-Ribose | + | + |
| D-Saccharate | + | + |
| L-Sérine | + | + |
| D-Sorbitol | + | + |
| L-Sorbose | + | + |
| Succinate | + | + |
| Sucrose | + | + |
| D-Tagatose | - | - |
| D-Tartrate | - | - |
| L-Tartrate* | - | + |
| méso-Tartrate | - | - |
| D-Tréhalose | + | + |
| Tricarballylate* | - | + |
| Trigonelline | - | - |
| Tryptamine | - | - |
| Tryptophane (couleur orange) | - | - |
| D-Turanose | - | - |
| L-Tyrosine | - | - |
| Xylitol* | + | - |
| D-xylose | + | + |

Croissance notée pour tous les substrats excepté l'esculine (couleur noire), l'hydroxyquinoléine-β-glucuronide (couleur noire), le tryptophane (couleur orange/rouille) et la L-histidine (couleur rose/rougeâtre) pour lesquels seul le développement de la coloration a été noté.
Symboles : +, croissance en 1-2 jours ; (+), croissance en 3-4 jours ; -, pas de croissance en 4 jours.

On voit que les souches BEC 1000 et ATCC 31646 donnent 11 réponses différentes sur 99, ce qui démontre que ces souches sont distinctes l'une de l'autre.

La souche BEC 1000 a été examinée du point de vue de sa pathogénicité et a été trouvée absolument dénuée de virulence pour l'homme.

La présente invention concerne aussi les souches mutantes obtenues à partir de la souche BEC 1000. Des souches mutantes utiles peuvent être obtenues par simple sélection de mutants spontanés isolés à partir d'une culture en fermenteur, ou bien en soumettant une culture de BEC 1000 à l'action d'un facteur mutagène, par exemple un rayonnement énergétique (rayons U.V., rayons X par exemple) ou un agent chimique [par exemple l'ozone, l'acide nitreux, la NTG(N-méthyl-N'-nitro-N-nitrosoguanidine) ou l'EMS (éthane-méthane-sulfonate)] en vue de tuer une portion importante des micro-organismes, en cultivant les micro-organismes et en sélectionnant ceux produisant davantage de polysaccharides.

La présente invention concerne enfin un procédé de production d'un polysaccharide présentant la structure chimique suivante : 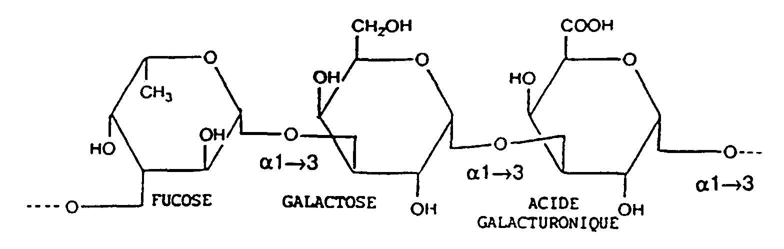 où certains des groupes hydroxyles sont acétylés, ce procédé comprenant (a) la croissance d'un microorganisme tel que défini à la revendication 1, dans un milieu nutritif aqueux par fermentation aérobie d'une source de glucide assimilable, puis (b) le recueil du polysaccharide à partir du moût obtenu à la fin de l'étape (a).

Le polysaccharide est produit par fermentation aérobie dans des conditions réglées d'un milieu nutritif aqueux approprié, inoculé avec la souche BEC 1000. Le milieu peut être tout milieu usuel contenant des sources de carbone, d'azote et des sels minéraux.

En général, des glucides (par exemple du glucose, du fructose, du maltose, du saccharose, du mannitol, de l'amidon, du sirop de maïs, du sorbitol, etc...) peuvent être utilisés isolément ou en combinaison comme sources de carbone assimilables dans le milieu nutritif. Une source de carbone préférée est le sorbitol. Typiquement, la quantité de glucides dans le milieu sera comprise entre environ 2 et 6% du poids du milieu.

Comme source d'azote, on peut utiliser diverses matières protéiques, par exemple de l'extrait de levure, de la farine de soja, des hydrolysats de protéines, de la liqueur de trempage du maïs, etc...). Typiquement, la source d'azote représentera entre 0,05 et 0,5% du poids du milieu.

Comme sels minéraux, on peut utiliser les sels habituellement employés dans les milieux nutritifs. Des exemples non limitatifs sont les phosphates, sulfates, chlorures et carbonates de sodium, potassium, ammonium, calcium et magnésium.

On peut conduire la fermentation à des températures de l'ordre de 25 à 35°C, de préférence de 28 à 32°C, à un pH d'environ 6,0 à 7,5, dans des conditions d'aération et d'agitation, pendant des durées de l'ordre de 2 à 4 jours.

La fermentation peut être effectuée dans un fermenteur classique en inoculant du milieu nutritif préalablement stérilisé, par exemple par chauffage à une température de l'ordre de 120°C ou par filtration stérilisante, à l'aide d'une culture de la souche BEC 1000.

A la fin de la période de fermentation, le moût de culture est soumis à un traitement protéolytique, puis autoclavé, refroidi, salé et acidifié. Le produit résultant est alors traité pour séparer le polysaccharide, par exemple par précipitation de ce dernier à l'aide d'un alcool (par exemple le méthanol, l'éthanol, le propanol ou l'isopropanol) ou autre non solvant (tel que l'acétone) du polysaccharide, puis purifié par essorage et lavage du précipité. Si désiré, on peut répéter plusieurs fois le processus de purification jusqu'à l'obtention d'un produit d'une pureté convenant à l'application envisagée.

Le produit purifié peut être mis sous forme de solution aqueuse, en présence d'un conservateur, pour l'utilisation, ou bien, peut être séché en une poudre, si désiré, pour le stockage.

L'exemple non limitatif suivant est donné en vue d'illustrer l'invention.

### Exemple : Production de polysaccharide contenant du L-fucose.

Un polysaccharide contenant du L-fucose est produit par fermentation sur un milieu de culture à base de sorbitol. Sa production se traduit par une importante élévation de la viscosité apparente du milieu. Cette viscosité constitue en fin de fermentation un élément limitant l'aération du milieu. La cinétique de croissance et de production du polysaccharide est liée à la consommation de sorbitol.

### PREPARATION DES INOCULUMS :

Milieu de culture :

| | |
|---|---|
| Néosorb® 70-07 (teneur en sorbitol : 70% M.S. ; vendu par ROQUETTE FRERES, Lille France) g/l | 17,90 g/l (soit 12,5 de sorbitol) |
| Peptone Biokar® 104003 (hydrolysat de protéines, vendu par SOLABIA-BIOKAR, Pantin, France) | 4,50 g/l |
| Extrait de levure | 0,05 g/l |
| KH₂PO₄ | 1,50 g/l |
| K₂HPO₄ | 4,50 g/l |
| MgSO₄, 7H₂O | 0,20 g/l |
| Pluronic® PE 61000 (agent anti-mousse, vendu par BASF, D-6700 Ludwigshafen, Allemagne) | 0,50 g/l |

Mise en solution dans de l'eau.
Condition de culture :
Stérilisation à 121°C pendant 30 minutes
Température de culture : 30°C
Taux d'inoculation : 5 à 10%
Aération : 1 VVM
pH non régulé (pH d'environ 7,00)
Durée de culture : 24 heures

### MILIEU DE PRODUCTION

Milieu de culture :

| | |
|---|---|
| Néosorb® 70-07 | 54,00 g/l (soit 38g/l de sorbitol) |
| Peptone Biokar® 104003 | 4,50 g/l |
| Extrait de levure | 0,05 g/l |
| KH₂PO₄ | 1,50 g/l |
| K₂HPO₄ | 4,50 g/l |
| MgSO₄, 7H₂O | 0,20 g/l |
| Pluronic® PE 61000 | 0,50 g/l |

Mise en solution dans de l'eau.
Conditions de culture (Fermenteur Chemap ayant un volume utile de 350 litres) :
Stérilisation à 120°C pendant 45 minutes
Température de culture : 30°C
Taux d'inoculation : environ 5%
Agitation : 300 rpm (agitateur de type Rushton)
Aération : 1 VVM
pH régulé à 7,0 par NaOH 7 N
Pression : 100 à 200 mbars
Durée de culture : 60 à 65 heures
Valeurs moyennes obtenues en production :

| | |
|---|---|
| Viscosité en fin de cycle | 40000 Mpa.s (Viscosimètre Brookfield DV-II+ modèle LV, mobile SP 31, chambre SC4-34/13R, 30°C) |
| Concentration du polysaccharide produit dans le milieu, calculée en L-fucose | 2 g/l (méthode de Dische et Shettles) |
| Sorbitol consommé | > 35 g/l (en sorbitol) |
| NaOH à 20% en poids consommée | 15 litres/m³ |
| Début de régulation du pH | 16 à 17 heures après inoculation du fermenteur |
| Extrait sec final du milieu de fermentation | ≈ 20 g/l |

Le pH peut très légèrement remonter en fin de fermentation (pH ≈ 7,2).

### RECUPERATION DU POLYSACCHARIDE

500 litres de moût de culture ont été ajustés à pH 8,2 avec de la soude, puis soumis à un traitement protéolytique à 50°C pendant 6 heures, à l'aide de 0,5 litre de Alcalase® (subtilisine A de *Bacillus licheniformis* vendue par la Société NOVO NORDISK, Danemark). Au bout des 6 heures, le pH a été réglé à 7,0 et on a autoclavé l'hydrolysat à 120°C, sous agitation, pendant 15 mn pour inactiver la charge microbienne, avant de le refroidir rapidement à 30°C et d'arrêter l'agitation. On ajoute alors 20 kg de chlorure de sodium sous agitation, on ajuste le pH à 4,5-5,0 avec du HCl, et on poursuit l'agitation pendant 15 mn pour bien dissoudre le sel. On ajoute au produit de l'éthanol à au moins 85° en une proportion correspondant à 1,1 fois le volume du produit, sous vigoureuse agitation. Un produit précipite qu'on laisse décanter pendant 4 h à 12 h après avoir coupé l'agitation. Le surnageant est filtré et renvoyé à un distillateur pour récupérer de l'éthanol en vue de son recyclage à l'étape de précipitation. Le précipité, imbibé d'un mélange eau-alcool, est essoré sur Büchner, agité dans une défloculeuse pneumatique pour éliminer les agglomérats, lavé avec un volume égal d'éthanol recyclé sous agitation, réessoré sur Büchner, réagité dans la défloculeuse, lavé avec de l'éthanol à 95°, agité et finalement essoré sur Büchner. Le précipité essoré obtenu est alors séché sur des plateaux dans une étuve à 40°C pendant 20 h. Le produit sec peut être stocké jusqu'à utilisation. En vue de celle-ci le produit est dissous, par exemple à raison d'environ 1% en poids, dans de l'eau sous agitation vigoureuse, de préférence avec addition d'un agent conservateur, par exemple du Phénonip® vendu par SIPCA (Paris, France)

Avantageusement, le pH de la solution est ajusté à environ 7,0.

Le produit finalement obtenu se présente sous la forme d'une solution visqueuse, opalescente, de très faible odeur, présentant une viscosité de 1000 + 200 Mpa.s. Ce produit est doté de qualités remarquables, à savoir :
. une teneur relativement élevée en L-fucose, substance dont le pouvoir anti-allergénique a fait l'objet de nombreux travaux ;
. des propriétés viscosantes ;
. une grande stabilité dans une large gamme de pH, de température et de salinité ;
. un pouvoir "auto-émulsionnant" ;
. un comportement rhéofluidifiant
qui le rendent éminemment approprié pour la formulation de produits cosmétiques tels que des produits pour le contour des yeux, des laits pour le corps, des produits pour peaux fragiles, des produits d'hygiène infantile, entre autres.

Il est à noter qu'on pourrait utiliser d'autres préparations enzymatiques commerciales équivalentes à l'Alcalase®.

## Revendications

1. Microorganisme du genre *Klebsiella pneumoniae,* subsp. *pneumoniae* déposé à la Collection Nationale de Cultures de Microorganismes sous le numéro I-1507 et ses mutants, capables de former un polysaccharide contenant du L-fucose, du D-galactose et de l'acide D-galacturonique.

2. Procédé de production d'un polysaccharide présentant la structure chimique suivante : 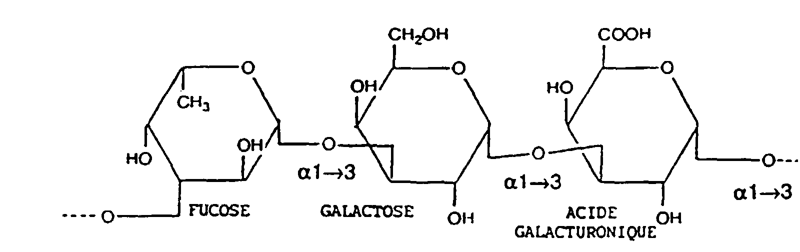 où certains des groupes hydroxyles sont acétylés, ce procédé comprenant (a) la croissance d'un microorganisme tel que défini à la revendication 1, dans un milieu nutritif aqueux par fermentation aérobie d'une source de glucide assimilable, puis (b) le recueil du polysaccharide à partir du moût obtenu à la fin de l'étape (a).

3. Procédé selon la revendication 2, caractérisé en ce que la source de glucide assimilable est du sorbitol.

4. Procédé selon la revendication 2 ou 3, caractérisé en ce que l'étape (b) comprend la soumission du moût de culture à un traitement d'hydrolyse des protéines par voie enzymatique.

## Claims

1. Microorganism of the genus *Klebsiella pneumonias* subsp. *pneumoniae,* deposited in the Collection Nationale de Cultures de Microorganismes with registration number I-1507, and mutants thereof, which can form a polysaccharide containing L-fucose, D-galactose and D-galacturonic acid.

2. Process for the production of a polysaccharide with the following chemical structure: 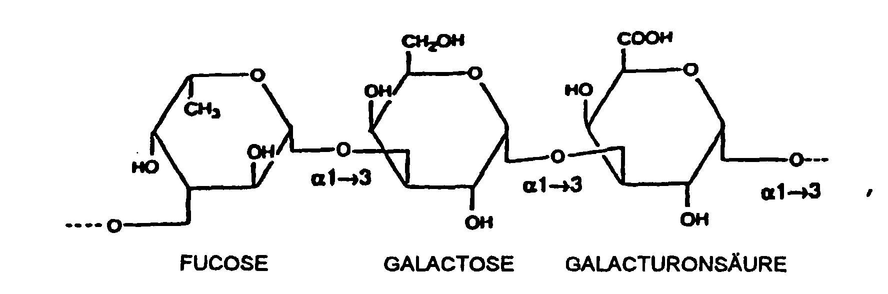 where some of the hydroxyl groups are acetylated, the process comprising (a) growing a microorganism as defined in claim 1 in an aqueous nutrient medium by aerobic fermentation of an assimilatable glucide source, then (b) recovering the polysaccharide from the suspension obtained at the end of step (a).

3. Process according to claim 2 characterised in that the source of assimilatable glucide is sorbitol.

4. Process according to claim 2 or 3 characterised in that step (b) comprises hydrolysing the proteins in the culture suspension using an enzymatic route.

## Patentansprüche

1. Mikroorganismus der Art *Klebsiella pneumoniae,* Unterart *pneumoniae,* hinterlegt bei der Collection Nationale de Cultures de Microorganismes unter der Nummer I-1507, und dessen Mutanten, die dazu in der Lage sind, ein L-Fucose, D-Galactose und D-Galacturonsäure enthaltendes Polysaccharid zu bilden.

2. Verfahren zur Herstellung eines Polysaccharids, das die folgende chemische Struktur aufweist: 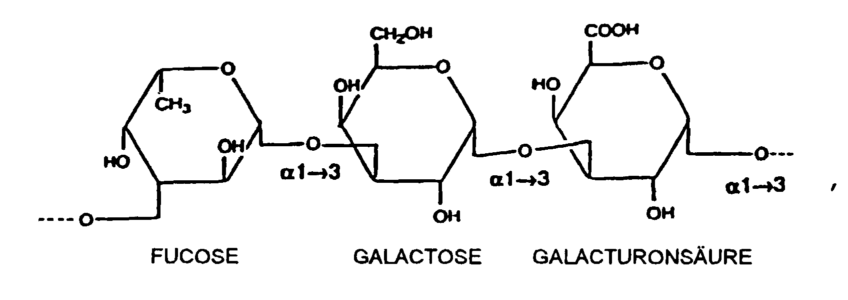 wobei einige Hydroxylgruppen acetyliert sind, wobei das Verfahren (a) das Wachstum eines gemäß Anspruch 1 definierten Mikroorganismus in einem wässrigen Nährmedium durch aerobe Fermentation einer Quelle assimilierbarer Kohlehydrate, anschließend (b) die Gewinnung von Polysaccharid aus dem am Ende von Stufe (a) erhaltenen Saft umfaßt.

3. Verfahren nach Anspruch 2, dadurch gekennzeichnet, dass die Quelle des assimilierbaren Kohlehydrats Sorbit ist.

4. Verfahren nach Anspruch 2 oder 3, dadurch gekennzeichnet, dass Stufe (b) das Anwenden einer enzymatischen Proteinhydrolysebehandlung auf den Saft der Kultur umfaßt.
